(19) Europäisches Patentamt
European Patent Office
Office européen des brevets

(11) **EP 4 353 155 A1**

(12) **EUROPEAN PATENT APPLICATION**

(43) Date of publication:
**17.04.2024 Bulletin 2024/16**

(21) Application number: **22213532.9**

(22) Date of filing: **14.12.2022**

(51) International Patent Classification (IPC):
**A61B 6/12** (2006.01)    **A61B 6/00** (2024.01)
**A61N 1/05** (2006.01)    **A61B 90/00** (2016.01)

(52) Cooperative Patent Classification (CPC):
**A61B 6/482; A61B 6/12; A61B 6/5217; A61N 1/05;**
A61B 6/5205; A61B 2090/3762

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.10.2022 US 202263415999 P**

(71) Applicant: **Koninklijke Philips N.V.**
**5656 AG Eindhoven (NL)**

(72) Inventors:
• **HAASE, Hans Christian**
**Eindhoven (NL)**

• **GRASS, Michael**
**Eindhoven (NL)**
• **SCHMITT, Holger**
**5656AG Eindhoven (NL)**
• **NICKISCH, Hannes**
**Eindhoven (NL)**
• **VAN DER HORST, Arjen**
**Eindhoven (NL)**
• **VEMBAR, Manindranath**
**Eindhoven (NL)**

(74) Representative: **Philips Intellectual Property & Standards**
**High Tech Campus 52**
**5656 AG Eindhoven (NL)**

(54) **PROVIDING GUIDANCE INFORMATION FOR AN IMPLANTABLE DEVICE EXTRACTION PROCEDURE**

(57)    A computer-implemented method of providing guidance information for an implantable device extraction procedure, is provided. The method includes: receiving (S 1 10) attenuation data (110) representing an implantable device (120) in an anatomical region, the attenuation data defining X-ray attenuation within the anatomical region; analyzing (S120) the attenuation data (110) to determine an amount of adhesion (130) between the implantable device (120) and a tissue (140) contacting the implantable device; and outputting (S130) guidance information for the implantable device extraction procedure based on the amount of adhesion (130).

FIG. 5

EP 4 353 155 A1

**Description**

TECHNICAL FIELD

The present disclosure relates to providing guidance information for an implantable device extraction procedure. A computer-implemented method, a computer program product, and a system, are disclosed.

BACKGROUND

Medical procedures often involve the implantation of devices into the body. For instance, the electrical leads of cardiac pacemakers are routinely implanted into the body in order to convey electrical pulses to the heart, and thereby regulate its beating. The leads are routed through the vasculature between the pacemaker that generates the electrical pulses, and cardiac tissue, where the distal ends of the leads deliver the electrical pulses. This allows the cardiac pacemaker to be placed in a more accessible location in the body, typically under a flap of skin in the chest.

The electrical leads of implantable cardioverter defibrillators, or "ICDs" are also routinely implanted into the body, in this case to convey electrical pulses from the ICD to the heart. The leads of ICDs are routed through the vasculature in a similar manner, in this case between the ICD that generates electrical pulses, and cardiac tissue, where the distal ends of the leads similarly deliver the electrical pulses. An ICD may operate in various modes. For instance, it may deliver a series of low-voltage impulses at a high rate to try to correct the heart rhythm, or it may deliver one or more small electrical shocks to the heart to try to restore the heart to a normal rhythm, or it may deliver one or more larger electrical shocks to the heart to try to restore the heart to a normal rhythm.

Aside from the electrical leads of cardiac pacemakers, and ICDs, other types of implantable devices may also be implanted into the body and used for other purposes, including for example an inferior vena cava "IVC" filter.

Occasionally, however, implantable devices may need to be extracted from the body. For instance, the electrical lead of a pacemaker, or an ICD, may need to be extracted in the event of its malfunction, or if an infection develops. If the lead has been in place for a short period of time, adhesion between the lead and any contacting tissue may be relatively weak. In this situation, it may be possible to extract the lead simply by pulling on the lead. However, if the lead has been in place for more than a few months, adhesion between the lead and contacting tissue may be relatively stronger. This relatively stronger adhesion, in combination with the tortuous path of the electrical lead through the vasculature, may make this technique unsuitable. In this situation various tools may be used to facilitate the extraction of the lead. For instance, a tool known as a locking stylet may be passed through a lumen in the lead, and used to provide extra support whilst pulling on the lead. The locking stylet transfers the force that is exerted by pulling on the lead, to the distal end of the lead, thereby facilitating its extraction. Alternatively, a tool that includes a sheath with a cutting blade at its distal end may be passed over the lead and used to separate the lead from the contacting tissue. An example of such a tool is the TightRail mechanical rotating dilator sheath that is marketed by Philips Healthcare, Best, The Netherlands. A variant of this type of tool includes a sheath with optical fibers instead of a cutting blade. In this case, the sheath is passed over the lead whilst the optical fibers irradiate tissue contacting the distal end of the sheath with laser light, thereby liberating the lead from the tissue. An example of such a tool is the GlideLight Laser sheath marketed by Philips Healthcare, Best, The Netherlands.

Thus, various techniques, and also tools, are available for use in extracting implantable devices such as electrical leads for pacemakers and ICDs, as well as other devices, from the body, should the need arise. However, it remains challenging to decide how to perform implantable device extraction procedures.

SUMMARY

According to one aspect of the present disclosure, a computer-implemented method of providing guidance information for an implantable device extraction procedure, is provided. The method includes:

receiving attenuation data representing an implantable device in an anatomical region, the attenuation data defining X-ray attenuation within the anatomical region;
analyzing the attenuation data to determine an amount of adhesion between the implantable device and a tissue contacting the implantable device; and
outputting guidance information for the implantable device extraction procedure based on the amount of adhesion.

In the above method, guidance information is outputted. The guidance information is based on the amount of adhesion between the implantable device and a tissue contacting the implantable device. The amount of adhesion is determined from attenuation data that defines X-ray attenuation within the anatomical region. Thus, the method provides a reliable, non-invasive technique for guiding the extraction of an implantable device.

Further aspects, features, and advantages of the present disclosure will become apparent from the following description of examples, which is made with reference to the accompanying drawings.

BRIEF DESCRIPTION OF THE DRAWINGS

Fig. 1 is an example of a projection X-ray image 110 representing a plurality of pacemaker leads $120_1$, $120_2$, in an anatomical region, in accordance with some aspects of the present disclosure.
Fig. 2 is a schematic diagram illustrating an example of a device 170 for use in an implantable device extraction procedure, in accordance with some aspects of the present disclosure.
Fig. 3 is a flowchart illustrating an example of a computer-implemented method of providing guidance information for an implantable device extraction procedure, in accordance with some aspects of the present disclosure.
Fig. 4 is a schematic diagram illustrating an example of a system 200 for providing guidance information for an implantable device extraction procedure, in accordance with some aspects of the present disclosure.
Fig. 5 is an example of a projection X-ray image 110 including guidance information for an implantable device extraction procedure in the form of an indication of the amount of adhesion 130 between an implantable device 120 and a tissue 140 contacting the implantable device, in accordance with some aspects of the present disclosure.
Fig. 6 is schematic diagram illustrating a display 220 including examples of guidance information 150, 160, 170, for an implantable device extraction procedure, in accordance with some aspects of the present disclosure.

DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to various examples of computer-implemented methods in which guidance information is provided for an implantable device extraction procedure. Reference is made to examples in which the implantable device is an electrical lead of a pacemaker. However, it is to be appreciated that the electrical lead serves only as an example, and that the methods disclosed herein may also be used to provide guidance information for extracting other types of implantable devices from the body. For instance, the methods disclosed herein may also be used to provide guidance information for extracting a lead of an ICD from the body, or for extracting the pacemaker, or the ICD, itself from the body, or for extracting an IVC filter, or for extracting a so-called leadless pacemaker or ICD from the body.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

As mentioned above, it remains challenging to decide how to perform implantable device extraction procedures.

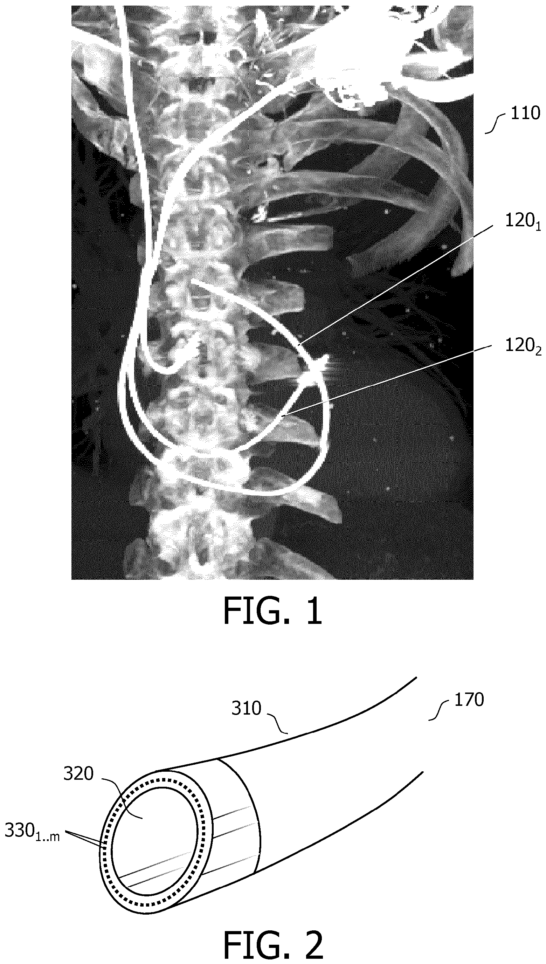

By way of an example, an implantable device extraction procedure is described in which the implantable device is an electrical lead of a pacemaker. Fig. 1 is an example of a projection X-ray image 110 representing a plurality of pacemaker leads $120_1$, $120_2$, in an anatomical region, in accordance with some aspects of the present disclosure. In the example illustrated in Fig. 1, the anatomical region is the cardiac region, and various ribs and vertebrae are visible. Two pacemaker leads $120_1$, and $120_2$ are also visible in Fig. 1. In general, a pacemaker may include one or more of such leads, and a distal end of the lead is implanted into cardiac tissue. In situations in which a single lead is used, the distal end of the lead is typically implanted into cardiac tissue in the right ventricle. If two leads are used, the distal end of one lead is often implanted into cardiac tissue in the right atrium, and the distal end of the other lead is often implanted into cardiac tissue in the right ventricle. When three leads are used, typically, the distal end of one lead is often implanted into cardiac tissue in the right atrium, the distal end of another lead is often implanted into cardiac tissue in the right ventricle, and the distal end of another lead is often implanted into cardiac tissue in the left ventricle.

As mentioned above, after the leads of a pacemaker have been implanted in the body, it may become necessary to extract a lead. For instance, the electrical lead of a pacemaker, or an ICD, may need to be extracted in the event of its malfunction, or if an infection develops. If the lead has been in place for a short period of time, adhesion between the lead and any contacting tissue may be relatively weak. In this situation, it may be possible to extract the lead simply by pulling on the lead. However, if the lead has been in place for more than a few months, adhesion between the lead and contacting tissue may be relatively stronger. For instance, over time, scar tissue may develop in the cardiac tissue around the distal end of the lead, and the lead can become strongly adhered to the scar tissue, hampering its extraction. Over time, the electrical lead can also adhere to tissue along the vessel wall in the vasculature through which the lead passes, and this also hampers its extraction. Over time, new tissue can also grow along the length of the lead, as described in a document by Keiler, J., et al., "Neointimal fibrotic lead encapsulation - Clinical challenges and demands for implantable cardiac electronic devices", Journal of Cardiology, Vol. 70, Issue 1, July 2017, Pages 7-17. Adhesion between the new tissue and the lead results in increased friction when removing the lead, hampering its extraction. The new tissue may also be attached to the vessel wall in the vasculature through which the lead passes, and this also hampers its extraction. Consequently, in these situations, the relatively stronger adhesion between the lead, and tissue contacting the lead, in combination with the tortuous path of the electrical lead through the vasculature (as seen in Fig. 1) may mean that the technique of extracting the lead simply by pulling on the lead, is unsuitable.

As mentioned above, various tools may be used to facilitate the extraction of a pacemaker lead in situations in which there is relatively stronger adhesion between the lead, and tissue contacting the lead. For instance, a tool known as a locking stylet may be used. Alternatively, a tool that includes a sheath with a cutting blade at its distal end may be passed over the lead and used to separate the lead from the contacting tissue. A variant of this type of tool includes a sheath with optical fibers instead of a cutting blade. In this case, the sheath is passed over the lead whilst the optical fibers irradiate tissue contacting the distal end of the sheath with laser light, thereby liberating the lead from the tissue. An example of this type of tool is illustrated in Fig. 2, which is a schematic diagram illustrating an example of a device 170 for use in an implantable device extraction procedure, in accordance with some aspects of the present disclosure.

The device 170 illustrated in Fig. 2 includes a sheath 310, with a bore 320. The distal ends of a plurality of the optical fibers $330_{1..m}$, are arranged around the bore. An optical source, not illustrated in Fig. 2, generates optical radiation, and the optical radiation is coupled to a proximal end of the optical fibers. In the example illustrated in Fig. 2, the optical source is an ultraviolet excimer laser with a wavelength of approximately 308 nm. In-use, the sheath 310 is passed over a pacemaker lead by passing the pacemaker lead through the bore 320 whilst the distal ends of the optical fibers $330_{1..m}$ irradiate the tissue surrounding the distal end of the sheath with the optical radiation from the laser. The effect of irradiating the tissue is to ablate tissue at a depth of approximately 50 microns, which liberates the pacemaker lead from the tissue.

However, it remains challenging to decide how to perform implantable device extraction procedures for such electrical leads, as well as other types of implantable devices. For instance, in the procedure described above, a physician needs to make decisions over which of the aforementioned techniques to use, and also which of the available tools to use. It may also be the case that different techniques and tools are appropriate for different stages of the extraction procedure. For instance, one technique may be appropriate for use in extracting the lead along its length, whereas another technique may be appropriate for extracting the distal end of the lead. Moreover, if a tool is to be used, the physician needs to decide which settings to use with the tool. Other types of implantable device extraction procedures face similar challenges.

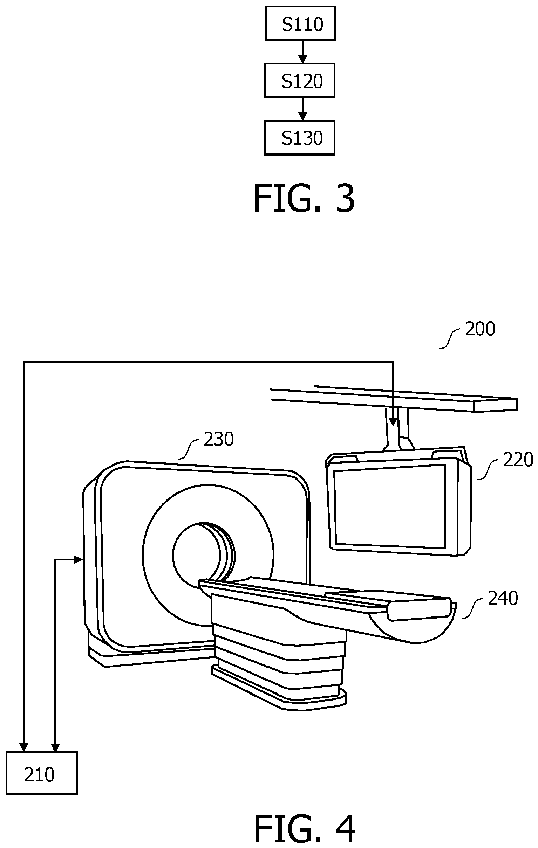

Fig. 3 is a flowchart illustrating an example of a computer-implemented method of providing guidance information for an implantable device extraction procedure, in accordance with some aspects of the present disclosure. Fig. 4 is a schematic diagram illustrating an example of a system 200 for providing guidance information for an implantable device extraction procedure, in accordance with some aspects of the present disclosure. The system 200 includes one or more processors 210. It is noted that operations described in relation to the method illustrated in Fig. 3, may also be performed by the one or more processors 210 of the system 200 illustrated in Fig. 4. Likewise, operations described in relation to the one or more processors 210 of the system 200, may also be performed in the method described with reference to Fig. 3. With reference to Fig. 3, the computer-implemented method of providing guidance information for an implantable device extraction procedure, includes:

receiving S 110 attenuation data 110 representing an implantable device 120 in an anatomical region, the attenuation data defining X-ray attenuation within the anatomical region;
analyzing S120 the attenuation data 110 to determine an amount of adhesion 130 between the implantable device 120 and a tissue 140 contacting the implantable device; and
outputting S 130 guidance information for the implantable device extraction procedure based on the amount of adhesion 130.

In the above method, guidance information is outputted. The guidance information is based on the amount of adhesion between the implantable device and a tissue contacting the implantable device. The amount of adhesion is determined from attenuation data that defines X-ray attenuation within the anatomical region. Thus, the method provides a reliable, non-invasive technique for guiding the extraction of an implantable device.

The above method is described in more detail below.

With reference to Fig. 3, in the operation S 110, attenuation data 110 is received. The attenuation data represents an implantable device 120 in an anatomical region, and defines X-ray attenuation within the anatomical region.

In general, the implantable device may be any type of implantable device, and the implantable device may be disposed in any anatomical region.

The attenuation data 110 that is received in the operation S 110 may in general represent a static image, or it may represent a temporal sequence of images. In the latter case, the temporal sequence of images may be generated substantially in real-time, and the method described above may be performed substantially in real-time. Consequently, live guidance may be provided for live attenuation data during an implantable device extraction procedure.

The attenuation data 110 that is received in the operation S 110 may in general be projection data, i.e. 2D data that can be used to generate a 2D image, or it may be volumetric data, i.e. 3D data that can be used to generate a 3D image. In the latter case, the data may be raw data, i.e. data that has not yet been reconstructed into a volumetric, or 3D image, or it may be image data, i.e. data that has already been reconstructed into a 3D image. Thus, in general, the attenuation data 110 that is received in the operation S110 may be generated by an X-ray projection imaging system, or by a CT imaging system.

A CT imaging system generates volumetric data by rotating, or stepping, an X-ray source-detector arrangement around an object and acquiring attenuation data for the object from multiple rotational angles with respect to the object. The volumetric data may then be reconstructed into a 3D image of the object. Examples of CT imaging systems include cone beam CT imaging systems, photon counting CT imaging systems, dark-field CT imaging systems, and phase contrast CT imaging systems. An example of a CT imaging system 230 that may be used to generate volumetric attenuation data 110 that is received in the operation S110, is illustrated in Fig. 4. By way of an example, the volumetric attenuation data 110 may be generated by the CT 5000 Ingenuity CT scanner that is marketed by Philips Healthcare, Best, The Netherlands.

As mentioned above, the attenuation data 110 that is received in the operation S110 may alternatively be generated by an X-ray projection imaging system. An X-ray projection imaging system typically includes a support arm such as a so-called "C-arm" that supports an X-ray source and an X-ray detector. X-ray projection imaging systems may alternatively include a support arm with a different shape to this example, such as an O-arm, for example. In contrast to a CT imaging system, an X-ray projection imaging system generates attenuation data for an object with the X-ray source and X-ray detector in a static position with respect to the object. The attenuation data, which may also be referred to as projection data, may then be used to generate a 2D image of the object. An example of an X-ray projection imaging system that may be used to generate projection attenuation data 110 that is received in the operation S110, is the Azurion 7 X-ray projection imaging system that is marketed by Philips Healthcare, Best, The Netherlands.

The attenuation data 110 that is received in the operation S 110 may alternatively be volumetric attenuation data that is generated by an X-ray projection imaging system. X-ray projection imaging systems may generate volumetric data by rotating, or stepping, their X-ray source and X-ray detector around an object and acquiring projection data for the object from multiple rotational angles with respect to the object. Image reconstruction techniques may then be used to reconstruct the projection data that is obtained from multiple rotational angles with respect to the object, into a volumetric image, and in a similar manner to the reconstruction of a volumetric image using attenuation data acquired from a CT imaging system. Volumetric data may therefore be generated by an X-ray projection imaging system. Thus, in examples in which volumetric attenuation data 110 is received in the operation S110, the volumetric attenuation data 110 may be generated by an X-ray projection imaging system.

In some examples, the attenuation data 110 that is received in the operation S110 is spectral attenuation data.

The spectral attenuation data defines the X-ray attenuation within the anatomical region in a plurality of different energy intervals $DE_{1..m}$. In general there may be two or more energy intervals; i.e. $m$ is an integer, and $m \geq 2$. The ability to generate X-ray attenuation data at multiple different energy intervals $DE_{1..m}$ distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable in conventional X-ray image data.

In examples in which the attenuation data that is received in the operation S110 is spectral attenuation data, it is noted that the spectral attenuation data may be provided in the form of projection data, or in the form of volumetric data. Thus, the spectral attenuation data that is received in the operation S110 may be generated by a spectral X-ray projection imaging system, or by a spectral CT imaging system. More generally, the spectral attenuation data that is received in the operation S110 may be generated by a spectral X-ray imaging system. Examples of spectral X-ray imaging systems that may be used to generate spectral attenuation data 110 that is received in the operation S110 include cone beam spectral X-ray imaging systems, photon counting spectral X-ray imaging systems, dark-field spectral X-ray imaging systems, and phase contrast spectral X-ray imaging systems. An example of a spectral CT imaging system that may be used to generate spectral attenuation data 110 that is received in the operation S110 is the Spectral CT 7500 that is marketed by Philips Healthcare, Best, The Netherlands.

In general, spectral attenuation data may be generated by various different configurations of spectral X-ray imaging systems that include an X-ray source and an X-ray detector. The X-ray source of a spectral X-ray imaging system may include multiple monochromatic sources, or one or more polychromatic sources, and the X-ray detector of a spectral X-ray imaging system may include: a common detector for detecting multiple different X-ray energy intervals, or multiple detectors wherein each detector detects a different X-ray energy interval $DE_{1..m}$, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and detectors may be used to detect X-rays within different X-ray energy intervals $DE_{1..m}$. In general, discrimination between different X-ray energy intervals may be provided at the source by temporally switching the X-ray tube potential of a single X-ray source, i.e. "rapid kVp switching", or by temporally switching, or filtering, the emission of X-rays from multiple X-ray sources. In such configurations, a common X-ray detector may be used to detect X-rays across multiple different energy intervals, attenuation data for each energy interval being generated in a time-sequential manner. Alternatively, discrimination between different X-ray energy intervals may be provided at the detector by using a multi-layer detector, or a photon counting detector. Such detectors can detect X-rays from multiple X-ray energy intervals $DE_{1..m}$ near-simultaneously, and thus there is no need to perform temporal switching at the source. Thus, a multi-layer detector, or a photon counting detector, may be used in combination with a polychromatic source to generate X-ray attenuation data at different X-ray energy intervals $DE_{1..m}$.

Other combinations of the aforementioned X-ray sources and detectors may also be used to provide spectral attenuation data. For example, in a yet further configuration, the need to sequentially switch different X-ray sources emitting X-rays at different energy intervals may be obviated by mounting X-ray source-detector pairs to a gantry at rotationally-offset positions around an axis of rotation. In this configuration, each source-detector pair operates independently, and separation between the spectral attenuation data for different energy intervals $DE_{1..m}$, is facilitated by virtue of the rotational offsets of the source-detector pairs. Improved separation between the spectral attenuation data for different energy intervals $DE_{1..m}$ may be achieved with this configuration by applying an energy-selective filter to the X-ray detector(s) in order to reduce the effects of X-ray scatter.

With reference to the example illustrated in Fig. 1, in this example, the attenuation data 110 that is received in the operation S110 represents an electrical lead $120_1$, $120_2$, of a pacemaker in the cardiac anatomical region. In this example, the attenuation data 110 comprises projection data, and is provided in the form of a projection X-ray image.

In general, the attenuation data 110 that is received in the operation S110 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. The attenuation data 110 that is received in the operation S110 may be received from various sources. For example, the attenuation data 110 may be received from an imaging system, such as one of the imaging systems described above. Alternatively, the attenuation data 110 may be received from another source, such as a computer readable storage medium, the Internet, or the Cloud, for example.

Returning to Fig. 3, in the operation S120, the attenuation data 110 is analyzed to determine an amount of adhesion 130 between the implantable device 120 and a tissue 140 contacting the implantable device.

As mentioned above, the extraction of implantable devices, such as the electrical lead of a pacemaker, can be hampered by adhesion between the lead and tissue contacting the lead. The adhesion may occur both along a length of the lead, as well as at a distal end of the lead. An insight exploited in the present disclosure is that a measure for this adhesion can be determined from the attenuation data 110 described above. Three approaches are described below for determining the amount of adhesion between the implantable device 120 and a tissue 140 contacting the implantable device, in the operation S120. In a first approach, the amount of adhesion 130 between the implantable device 120 and the tissue 140 contacting the implantable device is determined based on the Hounsfield Unit attenuation value of the tissue. In a second approach, spectral attenuation data is analyzed to determine the amount of adhesion 130 between the implantable device 120 and a tissue 140 contacting the implantable device. In a third approach, a neural network is used to predict the amount of adhesion 130 between the implantable device 120 and a tissue 140 contacting the implantable device. In the third approach, the attenuation data may be conventional X-ray attenuation data, or it may be spectral attenuation data. In any of these approaches, an operation of segmenting the received attenuation data 110 to identify the implantable device 120, may be performed prior to the analyzing S120 the attenuation data 110, in order to facilitate the analysis of the attenuation data. Various segmentation algorithms such as model-based segmentation, watershed-based segmentation, region growing or level sets or graphcuts may be used for this purpose. A neural network may also be trained to segment the attenuation data in order to identify the implantable device.

In the first approach, the operation of analyzing S120 the attenuation data 110 to determine an amount of adhesion 130 between the implantable device 120 and a tissue 140 contacting the implantable device, comprises:

determining a Hounsfield Unit attenuation value of the tissue 140 contacting the implantable device; and
determining the amount of adhesion 130 between the implantable device 120 and the tissue 140 contacting the implantable device based on the Hounsfield Unit attenuation value.

In this first approach, the attenuation data 110 may be volumetric data, i.e. 3D data. The volumetric data may be generated by a CT imaging system, or by rotating, or stepping, the X-ray source and X-ray detector of an X-ray projection imaging system around an anatomical region and acquiring projection data for the anatomical region from multiple rotational angles with respect to the anatomical region, as described above. The data may be conventional attenuation data, or spectral attenuation data, as also described above.

The Hounsfield Unit attenuation value is a relative quantitative measurement of radiodensity that is calculated based on a linear transformation of the linear attenuation coefficient of the X-ray beam. Distilled water, at standard temperature and pressure, is defined to be zero Hounsfield Units and air defined as -1000 HU. The Hounsfield Units attenuation value, *HU,* is calculated according to the Equation:

$$HU = 1000 \times \frac{\mu - \mu_{water}}{\mu_{water} - \mu_{air}} \qquad \text{Equation 1}$$

and wherein $\mu_{water}$ and $\mu_{air}$ represent the linear attenuation coefficients of water and air, respectively. Conventional CT imaging systems are routinely calibrated with water and air phantoms such that their attenuation data is inherently generated in Hounsfield Units. Consequently, the attenuation data 110 that is analyzed in the operation S120 may be the volumetric data that is generated by a conventional CT imaging system. Volumetric data that is generated by rotating, or stepping, the X-ray source and X-ray detector of an X-ray projection imaging system around an anatomical region, may also be calibrated in a similar manner. Thus, the attenuation data 110 that is analyzed in the operation S120 may alternatively be volumetric data that is generated by a conventional X-ray projection imaging system.

Attenuation data 110 may alternatively be provided in HU values by converting volumetric data that is generated by a spectral CT imaging system, or by converting volumetric data that is generated by rotating, or stepping, the X-ray source and X-ray detector of a spectral X-ray projection imaging system around an anatomical region. The spectral attenuation data that is generated by such imaging systems may be converted into Hounsfield Units, often referred-to as "synthetic" Hounsfield Units, using various techniques. One example of such a technique is disclosed in a document by Bornefalk, H., "Synthetic Hounsfield units from spectral CT data", Phys Med Biol., 2012 Apr 7;57(7):N83-7.

With reference to Fig. 1, in the first approach, voxels in the attenuation data 110 contacting the implantable device, e.g. the lead $120_1$ illustrated in Fig. 1, are identified, and the amount of adhesion 130 between the implantable device 120 and the tissue 140 contacting the implantable device, is determined based on the Hounsfield Unit attenuation values of the voxels. This operation may include segmenting the attenuation data 110 in order to identify the lead, as described above.

In the first approach, the operation of analyzing S120 the attenuation data 110 to determine an amount of adhesion 130 between the implantable device 120 and a tissue 140 contacting the implantable device, may include quantifying the amount of adhesion between the implantable device and a tissue contacting the implantable device with one or more adhesion classes, or adhesion scores. This may be performed by using a functional relationship between HU values and adhesion classes, or adhesion scores, to assign the voxels to adhesion classes, or adhesion scores. The functional relationship may be provided by a graph, or a lookup table, for example.

For instance, in one example, voxels contacting the implantable device that have relatively lower HU values are mapped to adhesion classes, or adhesion scores, representing a relatively low amount of adhesion, whereas voxels contacting the implantable device that have relatively higher HU values, are mapped to adhesion classes, or adhesion scores, representing a relatively higher amount of adhesion. For instance, uncoagulated blood is typically weakly adhered to a pacemaker lead, and consequently does not significantly hamper the extraction of the lead from the body. Uncoagulated blood typically has HU values from 30 HU to 45 HU. In this example, voxels contacting the lead that have HU values in this range are mapped adhesion classes, or adhesion scores, that represent a relatively low amount of adhesion. By contrast, clotted blood is typically more strongly adhered to the lead. Clotted blood may adhere to the pacemaker lead along its length, hampering its extraction by increasing friction between the lead and the surrounding vasculature. Clotted blood typically has HU values from 60 HU to 100 HU. Consequently, voxels contacting the lead that have HU values in this range would be mapped to adhesion classes, or adhesion scores, representing a relatively higher amount of adhesion. New tissue that has grown along the length of the pacemaker lead over time, and also scar tissue that has developed in the cardiac tissue around the distal end of the lead, is typically also more strongly adhered to the lead. New tissue, and also scar tissue, have relatively higher HU values than uncoagulated blood. New tissue, and also scar tissue, can hamper the extraction of the lead, as described above. Consequently, in the first approach, voxels contacting the lead that have HU values that are higher than that of uncoagulated blood, and which may also represent new tissue, or scar tissue, would also be mapped to adhesion classes or scores representing relatively higher amount of adhesion.

As mentioned, above, in a second approach, spectral attenuation data is analyzed to determine the amount of adhesion 130 between the implantable device 120 and a tissue 140 contacting the implantable device. In this approach, the attenuation data 110 comprises spectral attenuation data, and the spectral attenuation data defines the X-ray attenuation within the anatomical region in a plurality of different energy intervals $DE_{1..m}$.

In this second approach, the attenuation data 110 may be volumetric data, i.e. 3D data, or it may be projection data, i.e. 2D data.

With reference to Fig. 1, in the second approach, voxels, or pixels, in the attenuation data 110 contacting the implantable device, e.g. the lead $120_1$ illustrated in Fig. 1, are identified, and the amount of adhesion 130 between the implantable device 120 and the tissue 140 contacting the implantable device, is determined based on the effective atomic number, $Z_{eff}$, of the voxels/ pixels, or based on the type of the tissue represented by the voxels/ pixels. This operation may include segmenting the attenuation data 110 in order to identify the lead, as described above.

The effective atomic numbers of the voxels/ pixels provides an approximate indication of the strength of adhesion of a material represented by the voxels/ pixels, to the implantable device. Thus, voxels/ pixels that contact the implantable device that have a relatively low value of effective atomic number, tend to represent materials that are relatively weakly adhered to the implantable device. For instance, uncoagulated blood has a relatively low value of effective atomic number, and is typically weakly adhered to the implantable device. By contrast, voxels/ pixels that contact the implantable device that have a relatively higher value of effective atomic number, tend to represent materials that are relatively strongly adhered to the implantable device. For instance, coagulated blood, new tissue, and scar tissue, have a relatively higher value of effective atomic number, and are typically more strongly adhered to the implantable device. The effective atomic number may be calculated from the attenuation data 110 using various techniques. One example of a suitable technique is disclosed in the document by Saito, M., et al., "A simple formulation for deriving effective atomic numbers via electron density calibration from dual-energy CT data in the human body", Medical Physics, Vol. 44, Issue 6, June 2017, pages 2293-2303.

In the second approach, the operation of analyzing S120 the attenuation data 110 to determine an amount of adhesion 130 between the implantable device 120 and a tissue 140 contacting the implantable device, may include quantifying the amount of adhesion between the implantable device and a tissue contacting the implantable device with one or more adhesion classes, or adhesion scores. This may be performed by using a functional relationship between the effective atomic number, $Z_{eff}$, values, and adhesion classes, or adhesion scores, to assign the voxels/pixels to adhesion classes, or adhesion scores. The functional relationship may be provided by a graph, or a lookup table, for example.

For instance, in one example, voxels/ pixels contacting the implantable device that have relatively lower effective atomic number, $Z_{eff}$, values are mapped to adhesion classes, or adhesion scores, representing a relatively low amount of adhesion, whereas voxels/pixels contacting the implantable device that have relatively higher effective atomic number, $Z_{eff}$, values, are mapped to adhesion classes, or adhesion scores, representing a relatively higher amount of adhesion.

In the second approach, the amount of adhesion 130 between the implantable device 120 and the tissue 140 contacting the implantable device, may alternatively be determined based on the type of the tissue represented by the voxels/ pixels, rather than based on the effective atomic number, $Z_{eff}$, of the voxels/ pixels. In this case, the type of the tissue represented by the voxels/ pixels may be determined by applying a material decomposition algorithm to the spectral attenuation data.

In this approach, the type of the tissue contacting the implantable device may be used to reliably determine the amount of adhesion 130 between the implantable device 120 and the tissue 130 contacting the implantable device because different types of tissue are known to adhere to implantable devices with different strengths. In this approach, the operation of analyzing S120 the attenuation data 110 to determine an amount of adhesion 130 between the implantable device 120 and a tissue 140 contacting the implantable device, comprises:

applying a material decomposition algorithm to the spectral attenuation data to identify a type of the tissue contacting the implantable device; and
determining the amount of adhesion 130 between the implantable device 120 and the tissue 130 contacting the implantable device based on the type of the tissue.

In this approach, the operation of analyzing S120 the attenuation data 110 to determine an amount of adhesion 130 between the implantable device 120 and a tissue 140 contacting the implantable device, may include quantifying the amount of adhesion between the implantable device and a tissue contacting the implantable device with one or more adhesion classes, or adhesion scores.

A functional relationship between the type of the tissue and adhesion classes, or adhesion scores, may be used to assign the voxels/pixels in the attenuation data 110 to adhesion classes, or adhesion scores. The functional relationship may be provided by a graph, or a lookup table, for example.

For instance, in one example, voxels/ pixels contacting the implantable device representing the tissue type "soft tissue" are mapped to adhesion classes, or adhesion scores, representing a relatively low amount of adhesion, whereas voxels/pixels contacting the implantable device that represent the tissue type "dense tissue" are mapped to adhesion classes, or adhesion scores, representing a relatively higher amount of adhesion.

Various material decomposition algorithms may be used to identify the type of a tissue, in accordance with this example. One example of a material decomposition algorithm that may be used is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another example of a material decomposition algorithm that may be used is disclosed in a document by Roessl, E. and Proksa, R., "K-edge imaging in X-ray computed tomography using multi-bin photon counting detectors", Phys Med Biol. 2007 Aug 7, 52(15): 4679-96. Another example of a material decomposition algorithm that may be used is disclosed in the published PCT patent application WO/2007/034359 A2. Another example of a material decomposition algorithm that may be used is disclosed in a document by Silva, A. C., et al., "Dual-energy (spectral) CT: applications in abdominal imaging", Radio-Graphics 2011; 31(4):1031-1046.

As mentioned above, in a third approach, a neural network is used to predict the amount of adhesion 130 between the implantable device 120 and a tissue 140 contacting the implantable device. In this approach, the operation of analyzing S120 the attenuation data 110 to determine an amount of adhesion 130 between the implantable device 120 and a tissue 140 contacting the implantable device, comprises:

inputting the received attenuation data 110 into a neural network; and
predicting, using the neural network, the amount of adhesion 130 between the implantable device 120 and a tissue 140 contacting the implantable device; and
wherein the neural network is trained to predict the amount of adhesion between the implantable device and a tissue contacting the implantable device using training attenuation data comprising a plurality of training images representing an implantable device in an anatomical region, and ground truth data comprising ground truth adhesion values corresponding to the training images, the ground truth adhesion values representing the amount of adhesion between the implantable device and a tissue contacting the implantable device.

In this approach, the attenuation data that is inputted into the neural network may be conventional X-ray attenuation data, or it may be spectral attenuation data. The attenuation data may be volumetric data, or it may be projection data. The attenuation data may represent a static image, or it may represent a temporal sequence of images. The neural network may be provided by various architectures, such as for example a convolutional neural network, "CNN", a recurrent neural network "RNN", or a transformer, and so forth.

In one example, the neural network is trained to predict the amount of adhesion 130 between the implantable device 120 and a tissue 140 contacting the implantable device based further on one or more of: age, gender, comorbidity, and age of the implantable device. The use of such additional information improves the neural network's predictions.

The neural network may be trained to predict the amount of adhesion between the implantable device, and a tissue contacting the implantable device, by:

receiving the training attenuation data;

inputting the training attenuation data into the neural network; and
for each of a plurality of the training images:
predicting a value for an amount of adhesion between the implantable device and a tissue contacting the implantable device, using the neural network;
adjusting parameters of the neural network based on a difference between the predicted value for the amount of adhesion and the ground truth adhesion value; and
repeating the predicting, and the adjusting, until a stopping criterion is met.

In this example, the training attenuation data comprises a plurality of training images representing an implantable device in an anatomical region, and ground truth data comprising ground truth adhesion values corresponding to the training images, the ground truth values representing the amount of adhesion between the implantable device and a tissue contacting the implantable device.

In this example, the training attenuation data defines X-ray attenuation within an anatomical region. The training attenuation data includes training images that represent the same type of implantable device, e.g. a pacemaker lead, as that for which the predictions are made by the neural network. The training attenuation data may be generated by the same type of X-ray imaging system, i.e. a volumetric/ projection/ spectral imaging system, as the attenuation data 110 for which the predictions are made by the neural network. Alternatively, the training attenuation data may be pseudo attenuation data that is generated by a different type of imaging system, and which is processed in order to resemble data generated by the type of X-ray imaging system for which the predictions are made by the neural network. For instance, volumetric "CT" images may be projected in order to resemble projection X-ray images. The training images may be curated from records of historic implantable device extraction procedures. The training data may be generated from some tens, hundreds, or thousands of such procedures, and the training data may represent subjects with a range of different ages, different genders, and different body mass index "BMI" values.

The corresponding ground truth data may be generated automatically, or by experts. The ground truth data may be determined automatically by quantifying the amount of adhesion between the implantable device and a tissue contacting the implantable device with adhesion classes, or adhesion scores, using the techniques described above. Alternatively, experts may label regions of the images in the training attenuation data with the ground truth values representing the amount of adhesion between the implantable device and a tissue contacting the implantable device.

If additional information such as age, gender, comorbidity, and age of the implantable device, is inputted into the neural network in order to generate its predictions, corresponding values for this information is also inputted into the neural network during its training.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In any of the aforementioned three approaches for determining the amount of adhesion between the implantable device and a tissue contacting the implantable device, the amount of adhesion may be determined for one or more positions on a surface of the device. For instance, in the example of a pacemaker lead, the amount of adhesion may be determined at a distal end of the lead, or for multiple positions on the surface of the distal end of the pacemaker lead. Some examples of implantable devices, such as a pacemaker lead, have an elongate shape, and the adhesion at positions along a length of the device is relevant to determining guidance information for extracting the implantable device. Thus, in one example, the operation of analyzing S 120 the attenuation data 110 to determine an amount of adhesion 130 between the implantable device 120 and a tissue 140 contacting the implantable device, is performed for a plurality of positions along a length of the implantable device.

In a related example, a total amount of adhesion may be determined between the implantable device and a tissue contacting the implantable device. In this example, the method described with reference to Fig. 3 includes determining a total amount of adhesion between the implantable device 120 and a tissue 140 contacting the implantable device, based on the amount of adhesion 130 between the implantable device 120 and the tissue 140 contacting the implantable device at the plurality of positions along the implantable device; and the outputting S 130 guidance information for the implantable device extraction procedure, is based on the total amount of adhesion.

In this example, the total amount of adhesion may be determined by integrating the amount of adhesion at the plurality of positions along the implantable device, for example. The total amount of adhesion provides an indicator for the overall complexity of the implantable device extraction procedure.

Returning to Fig. 3, in the operation S 130, guidance information is outputted. This operation is performed for each of the aforementioned three approaches for determining the amount of adhesion between the implantable device and a tissue contacting the implantable device. Various types of guidance information may be outputted in the operation S 130, and the guidance information may be outputted in various ways. In some examples, the guidance information is outputted in a visual format. For instance, in one example, the guidance information is outputted to a display. The guidance information may be outputted to the display 220 illustrated in Fig. 4, for example. The guidance information may alternatively be outputted in other ways, including audially, or to a printer, or to a computer readable storage medium.

As mentioned above, various types of guidance information may be outputted in the operation S130. By way of some examples, the outputting S130 guidance information for the implantable device extraction procedure, may include outputting an indication of one or more of:

the amount of adhesion 130 between the implantable device 120 and a tissue 140 contacting the implantable device;
an expected complexity of the implantable device extraction procedure;
an expected duration 160 of the implantable device extraction procedure;
an outcome metric for the implantable device extraction procedure;
a recommended device 170 for use in the implantable device extraction procedure;
a recommended device setting for use in the implantable device extraction procedure;
a recommended procedural step for use in the implantable device extraction procedure.

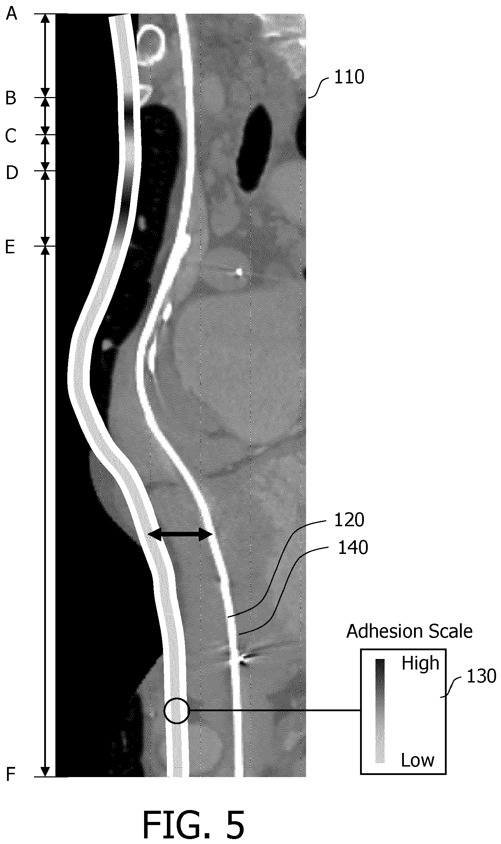

Fig. 5 is an example of a projection X-ray image 110 including guidance information for an implantable device extraction procedure in the form of an indication of the amount of adhesion 130 between an implantable device 120 and a tissue 140 contacting the implantable device, in accordance with some aspects of the present disclosure.

In the example illustrated in Fig. 5, the implantable device is a pacemaker lead, and the guidance information is overlaid onto a projection X-ray 110. In the illustrated example, the projection X-ray image 110 is generated from the attenuation data 110 that is received in the operation S110. However, the projection X-ray image 110 may alternatively be generated from other types of attenuation data represent the implantable device in the anatomical region. For instance, volumetric image data may be projected in order to provide a projected image, and onto which the guidance information is overlaid.

In the example illustrated in Fig. 5, the guidance information is overlaid on the projection X-ray image 110 adjacent to the pacemaker lead, and the guidance information includes an indication of the amount of adhesion 130 between the pacemaker lead 120 and a tissue 140 contacting the implantable device. In Fig. 5, the amount of adhesion is indicated at multiple positions along a length of the pacemaker lead. In this example, the guidance information informs a physician of sections of the pacemaker lead that may be difficult to extract. With reference to the Adhesion Scale illustrated in Fig. 5, in the illustrated example, a relatively higher amount adhesion between the pacemaker lead and the vessel wall is indicated for the sections B - C, and D - E, than for the sections A - B, C - D, and E - F. A physician may use this guidance information to e.g. take additional time in the extraction of the lead for sections B - C, and D - E. Based on this information, the physician may also decide to select a specific device for these sections, such as a sheath with a cutting blade or a laser at its distal end, rather than a locking stylet, in order to extract the pacemaker lead.

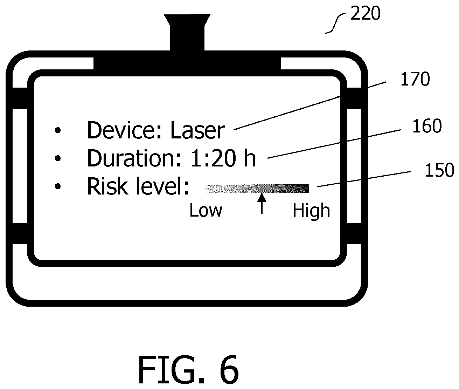

As mentioned above, other types of guidance information may alternatively, or additionally, be outputted in the operation S130. Fig. 6 is schematic diagram illustrating a display 220 including examples of guidance information 150, 160, 170, for an implantable device extraction procedure, in accordance with some aspects of the present disclosure.

In one example, the operation of outputting S 130 guidance information for the implantable device extraction procedure, comprises outputting an indication of an expected complexity of the implantable device extraction procedure.

The expected complexity of the implantable device extraction procedure may be determined based on the amount of adhesion 130 using a functional relationship. For instance, a functional relationship between the expected complexity and the amount of adhesion 130 may be used to assign an expected complexity to portions of the implantable device, or to the entire implantable device. The functional relationship may be provided by a graph, or a lookup table, or a decision tree, for example. For example, an indication of the expected complexity for the entire implantable device may be obtained by mapping the average value of the amount of adhesion 130 along a length of the pacemaker lead, to a complexity scale of 1 to 10, using a functional relationship in the form of a graph.

Alternatively, the expected complexity of the implantable device extraction procedure may be determined based on the amount of adhesion 130 using the neural network that predicts the amount of adhesion between the implantable device and a tissue contacting the implantable device. In this case, the neural network may use the predicted amount of adhesion between the implantable device and a tissue contacting the implantable device, as an input to further predict the complexity. The neural network may alternatively directly predict the complexity, in which case the complexity is implicitly predicted based on the amount of adhesion since the neural network is trained using training attenuation data that includes ground truth values representing the amount of adhesion between the implantable device and a tissue contacting the implantable device. If a neural network is used to determine the expected complexity of the implantable device extraction procedure, additional training data in the form of a ground truth value for the complexity, is also used to train the neural network. The ground truth value may be provided by an expert, for instance by the physician that performed the historic procedure from which the training attenuation data was curated.

In another example, the operation of outputting S130 guidance information for the implantable device extraction procedure, comprises outputting an indication of an expected duration 160 of the implantable device extraction procedure. The expected duration 160 of the implantable device extraction procedure may be determined in a similar manner as the expected complexity. In other words, in one example, the expected duration 160 of the implantable device extraction procedure may be determined based on the amount of adhesion 130 using a functional relationship. For example, an indication of the expected duration 160 may be obtained by mapping an integrated value for the amount of adhesion 130 along a length of the pacemaker lead, to a procedure duration time, using a functional relationship in the form of a graph.

Alternatively, the expected duration 160 may be determined based on the amount of adhesion 130 using the neural network that predicts the amount of adhesion between the implantable device and a tissue contacting the implantable device. In this case, the neural network may use the predicted amount of adhesion between the implantable device and a tissue contacting the implantable device, as an input to further predict the expected duration 160. The neural network may alternatively directly predict the expected duration 160, in which case the expected duration 160 is implicitly predicted based on the amount of adhesion since the neural network is trained using training attenuation data that includes ground truth values representing the amount of adhesion between the implantable device and a tissue contacting the implantable device. If a neural network is used to determine the expected duration 160, additional training data in the form of a ground truth value for the duration, is also used to train the neural network. The ground truth value may be known from the historic procedure from which the training attenuation data was curated.

In another example, the operation of outputting S130 guidance information for the implantable device extraction procedure, comprises outputting an indication of an outcome metric for the implantable device extraction procedure. The outcome metric may represent various factors. Examples of outcome metrics that may be outputted in this example include a probability of success or failure of the procedure, or the probability of a medical complication arising from the procedure. The outcome metric may be determined in a similar manner as the expected complexity. In other words, in one example, the outcome metric may be determined based on the amount of adhesion 130 using a functional relationship. For example, an indication of the outcome metric may be obtained by mapping the maximum value for the amount of adhesion 130 along a length of the pacemaker lead, to an outcome metric, using a functional relationship in the form of a lookup table. The maximum value may represent the most challenging part of the extraction procedure, and may therefore be an indicator of the outcome.

Alternatively, the outcome metric may be determined based on the amount of adhesion 130 using the neural network that predicts the amount of adhesion between the implantable device and a tissue contacting the implantable device. In this case, the neural network may use the predicted amount of adhesion between the implantable device and a tissue contacting the implantable device, as an input to further predict the outcome metric. The neural network may alternatively directly predict the outcome metric, in which case the outcome metric is implicitly predicted based on the amount of adhesion since the neural network is trained using training attenuation data that includes ground truth values representing the amount of adhesion between the implantable device and a tissue contacting the implantable device. If a neural network is used to determine the outcome metric, additional training data in the form of a ground truth value for the outcome metric, is also used to train the neural network. The ground truth value may be known from the historic procedure from which the training attenuation data was curated.

In another example, the operation of outputting S130 guidance information for the implantable device extraction procedure, comprises outputting an indication of a recommended device 170 for use in the implantable device extraction procedure. Examples of recommended devices that may be outputted in this example include a recommendation to use the laser device illustrated in Fig. 2, or the mechanical rotating dilator sheath described above. The recommended device 170 may be determined in a similar manner as the expected complexity. In other words, in one example, the recommended device 170 may be determined based on the amount of adhesion 130 using a functional relationship. For example, an indication of the recommended device 170 may be obtained by mapping the maximum value for the amount of adhesion 130 along a length of the pacemaker lead, or the total length of the pacemaker lead for which the amount of adhesion 130 exceeds a threshold value, to a recommended device 170, using a functional relationship in the form of a lookup table, or a decision tree.

Alternatively, the recommended device 170 may be determined based on the amount of adhesion 130 using the neural network that predicts the amount of adhesion between the implantable device and a tissue contacting the implantable device. In this case, the neural network may use the predicted amount of adhesion between the implantable device and a tissue contacting the implantable device, as an input to further predict the recommended device 170. The neural network may alternatively directly predict the recommended device 170, in which case the recommended device 170 is implicitly predicted based on the amount of adhesion since the neural network is trained using training attenuation data that includes ground truth values representing the amount of adhesion between the implantable device and a tissue contacting the implantable device. If a neural network is used to determine the recommended device 170, additional training data in the form of a ground truth value for the recommended device 170, is also used to train the neural network. The ground truth value may be known from the historic procedure from which the training attenuation data was curated.

In another example, the operation of outputting S130 guidance information for the implantable device extraction procedure, comprises outputting an indication of a recommended device setting for use in the implantable device extraction procedure. Examples of recommended device settings that may be outputted in this example include a power level of the laser of the device illustrated in Fig. 2, or a rotation speed of the mechanical rotating dilator sheath described above. The recommended device setting may be determined in a similar manner as the expected complexity. In other words, in one example, recommended device setting may be determined based on the amount of adhesion 130 using a functional relationship. For example, an indication of the recommended device setting may be obtained by mapping the amount of adhesion 130 along a length of the pacemaker lead, to recommended device settings, using a functional relationship in the form of a graph.

Alternatively, the recommended device setting may be determined based on the amount of adhesion 130 using the neural network that predicts the amount of adhesion between the implantable device and a tissue contacting the implantable device. In this case, the neural network may use the predicted amount of adhesion between the implantable device and a tissue contacting the implantable device, as an input to further predict the recommended device setting. The neural network may alternatively directly predict the recommended device setting, in which case the recommended device setting is implicitly predicted based on the amount of adhesion since the neural network is trained using training attenuation data that includes ground truth values representing the amount of adhesion between the implantable device and a tissue contacting the implantable device. If a neural network is used to determine the recommended device setting, additional training data in the form of a ground truth value for the recommended device setting, is also used to train the neural network. The ground truth value may be known from the historic procedure from which the training attenuation data was curated.

In another example, the operation of outputting S130 guidance information for the implantable device extraction procedure, comprises outputting an indication of a recommended procedural step for use in the implantable device extraction procedure. Examples of recommended procedural steps that may be outputted in this example include "advance extraction device at (a specified) rate", "withdraw extraction device 5 centimeters, rotate through (a specified) angle, then re-advance tool", The recommended procedural step may be determined in a similar manner as the recommended device setting. In other words, in one example, the recommended procedural step may be determined based on the amount of adhesion 130 using a functional relationship. For example, an indication of the rate of advancement of the extraction device may be obtained by mapping the values of the amount of adhesion 130 along a length of the pacemaker lead, to recommended advancement rates, using a functional relationship in the form of a graph.

Alternatively, the recommended procedural step may be determined based on the amount of adhesion 130 using the neural network that predicts the amount of adhesion between the implantable device and a tissue contacting the implantable device. In this case, the neural network may use the predicted amount of adhesion between the implantable device and a tissue contacting the implantable device, as an input to further predict the recommended procedural step. The neural network may alternatively directly predict the recommended procedural step, in which case the recommended procedural step is implicitly predicted based on the amount of adhesion since the neural network is trained using training attenuation data that includes ground truth values representing the amount of adhesion between the implantable device and a tissue contacting the implantable device. If a neural network is used to determine the recommended procedural step, additional training data in the form of a ground truth value for the recommended procedural step, is also used to train the neural network. The ground truth value may be known from the historic procedure from which the training attenuation data was curated.

Other types of guidance information may also be provided. In another example, an indication of the value of a risk metric is outputted. In this example, the method described with reference to Fig. 3 includes:

analyzing the attenuation data 110 to determine a value of a risk metric 150, the value of the risk metric representing a risk of damage to a tissue represented in the attenuation data 110 and/or a risk of fracturing the implantable device; and

wherein the outputting S130 guidance information for the implantable device extraction procedure based on the amount of adhesion 130, comprises outputting an indication of the value of the risk metric 150.

In this example, the tissue represented in the attenuation data 110 may be the lung, or a heart valve, or a vessel wall, for example. The value of the risk metric representing a risk of damage to the tissue represented in the attenuation data 110 may be determined based on a type of the tissue surrounding the implantable device 120, and a distance between the implantable device and the tissue, for example. In this example, a list of tissue types may be identified and labelled with a degree of sensitivity to damage. The value of the risk metric may be determined by dividing the degree of sensitivity to damage by the distance to the tissue. The distance may be measured in an image that is generated from the attenuation data 110. For example, the distance may be measured in a projection X-ray image, or in a volumetric X-ray image. Thus, nearby tissues that are deemed to be highly sensitivity to damage, are associated with a high risk metric. In one example, one or more organs-at-risk, such as a lung, are identified in the attenuation data 110 using segmentation techniques. If a tissue represented in the attenuation data 110 is located between the implantable device and the organ at risk, a relatively higher value of the risk metric 150 may be assigned to the tissue than if the implantable device is located between the tissue and the organ at risk. Consequently, the risk of damage to e.g. a vessel wall may be deemed higher for a portion of the vessel wall that is on the side of the pacemaker lead that faces the lung, than for a portion of the vessel wall that is on the opposite side of the pacemaker lead. The tissue types may be identified by segmenting the attenuation data 110 and labelling the tissue types using an anatomical atlas, for example. Alternatively, if the attenuation data 110 is spectral attenuation data, different tissue types may be identified by applying a material decomposition algorithm to the attenuation data 110. The risk metric may also be predicted using the neural network described above. In this case, additional training data in the form of a ground truth value(s) for the risk metric, is also used to train the neural network. For instance, an expert may label the training images in the training data with ground truth value(s) for the risk metric.

In this example, the value of a risk metric representing the risk of fracturing the implantable device may alternatively be determined. Over time, an implantable device such as a pacemaker lead may become weak, and is consequently susceptible to fracture during its removal. In this case, the risk metric may be determined from the attenuation data by identifying cracks in the implantable device and/or significant tortuosity along a length of the implantable device, for example. The value of the risk metric may also be predicted using the neural network described above. In this case, additional training data in the form of a ground truth value(s) for the risk metric, is also used to train the neural network. For instance, an expert may label portions of implantable devices in the training data that have fractured during their removal.

In another example, implantable device-induced inflammation in the anatomical region is identified. The identification of such inflammation is relevant to implantable device extraction procedures because it informs a physician about the regions in which additional care needs to be taken during extraction, as well as the urgency of the procedure. In the example of a pacemaker lead, it may also inform the point up to which the lead needs to be extracted.

In this example, the method described with reference to Fig. 3 includes:

analyzing the attenuation data 110 to determine a presence of implantable device-induced inflammation in the anatomical region; and

outputting an indication of the presence of the implantable device-induced inflammation.

In this example, the presence of the implantable device-induced inflammation may be determined based on measurements made in an image that is generated from the attenuation data. For instance, inflammation may be determined based on pathological observations such a swollen intima or mural thickening with increased X-ray attenuation. Image-based measurements of the sizes of such regions may be compared to reference values in order to determine the presence of such inflammation. In examples in which the attenuation data 110 includes spectral attenuation data, a so-called "$Z_{eff}$ image", i.e. an image derived from spectral CT data that represents the effective atomic number, may also be generated and used to identify inflammation based on the presence of regions that have anomalous high values of effective atomic number.

In another example, the method described with reference to Fig. 3 includes:

analyzing the attenuation data 110 to determine a stability metric for the implantable device; and
outputting an indication of the stability metric.

In this example, the stability metric represents the mechanical stability of the implantable device. In the example of a pacemaker lead, the mechanical stability of the lead is impacted by cracks in the lead, as described above, and also by the strength of the attachment of the lead to cardiac tissue at its distal end. The mechanical stability affects the probability of the extraction procedure succeeding, and also the choice of extraction device and technique. The stability metric may be determined by analyzing an image generated from the attenuation data 110 to identify such cracks, as described above, or alternatively by determining a depth of insertion of the distal end of the pacemaker lead into cardiac tissue. For example, pacemaker leads that have cracks, or which are only marginally inserted into cardiac tissue may be deemed unstable. Similarly, an IVC filter may have cracks around its circumference. The stability metric may be used by the physician to determine at which positions along the length of the pacemaker lead, or at which positions around an IVC filter, that additional care should be taken.

In some situations, different techniques, or different extraction tools, may be better suited to removing different sections of the pacemaker lead. For instance, a rotating dilator sheath may be better suited to removing a length of the pacemaker lead along the vasculature, whereas a locking stylet may be better suited for removing the distal end of the pacemaker lead from cardiac tissue. In other situations, pacemaker leads that are difficult to extract may be abandoned. For example, if the risks of their removal outweigh the advantages, pacemaker leads may be abandoned entirely, or they may be cut along their length such that a portion of the lead is abandoned in the body. In such situations it is useful to recommended a length of the pacemaker lead that should be extracted. In one example, the method described with reference to Fig. 3 includes:

calculating a recommended length of the implantable device 120 to be extracted in the implantable device extraction procedure, the recommended length being calculated based on the amount of adhesion 130 between the implantable device 120 and a tissue 140 contacting the implantable device and/or based on the stability metric and/or the presence of the implantable device-induced inflammation; and
outputting an indication of the recommended length.

In this example, different devices may be recommended for removal of the implantable device based on the amount of adhesion 130. For instance, if the amount of adhesion between the implantable device and the contacting tissue is low, a single tool may be recommended for extraction of the entire device. By contrast, if the amount of adhesion 130 along the length of the pacemaker lead is relatively low, and the amount of adhesion 130 at a distal end of the pacemaker lead is relatively higher, a first tool may be recommended for extraction of the length of the pacemaker lead, and a second tool recommended for extraction of the distal end. Alternatively, sections of the pacemaker lead for which the adhesion 130 between the implantable device 120 and a tissue 140 contacting the implantable device is weak, or moderate, may be recommended for extraction, whereas sections of the pacemaker lead for which the adhesion 130 between the implantable device 120 and a tissue 140 contacting the implantable device is strong may be recommended for abandonment. The recommended length of the implantable device may be indicated as an overlay on an image of the implantable device, for example. The stability metric and the presence of inflammation may also be factors that influence the recommended length of the lead for extraction. For instance it may be recommended to remove a portion of a lead that is close to an inflammation, or it may be recommended not to remove a portion of a lead if the lead is deemed unstable.

In one example, the outputted amount of adhesion 130 between the implantable device 120 and a tissue 140 contacting the implantable device, is used to generate control signals for a medical robot to perform the implantable device extraction procedure. The amount of adhesion 130 may be used to generate control signals for the navigation and/or the actuation of an interventional tool that is controlled by the medical robot. The interventional tool may for instance be the aforementioned TightRail mechanical rotating dilator sheath, or the aforementioned GlideLight Laser sheath. The control signals may for instance navigate the interventional tool to a region of tissue wherein the amount of adhesion 130 between the implantable device 120 and a tissue 140 contacting the implantable device exceeds a threshold value. The control signals may for instance control an actuation of the interventional tool such that the interventional tool separates the implantable device from tissue contacting the implantable device. The control signals may provide a relatively higher degree of actuation in regions in which the tissue has a relatively higher amount of adhesion 130 between the implantable device 120 and a tissue 140 contacting the implantable device, than in regions in which the tissue has a relatively lower amount of adhesion 130 between the implantable device 120 and a tissue 140 contacting the implantable device. The magnitude of the actuation may also be modulated based on value of the risk metric, in order to thereby reduce the risk of damage to tissue.

In another example, a computer program product is provided. The computer program product comprises instructions which when executed by one or more processors, cause the one or more processors to carry out a method of providing guidance information for an implantable device extraction procedure. The method comprises:

receiving S 110 attenuation data 110 representing an implantable device 120 in an anatomical region, the attenuation data defining X-ray attenuation within the anatomical region;
analyzing S 120 the attenuation data 110 to determine an amount of adhesion 130 between the implantable device 120 and a tissue 140 contacting the implantable device; and
outputting S 130 guidance information for the implantable device extraction procedure based on the amount of adhesion 130.

In another example, a system 200 for providing guidance information for an implantable device extraction procedure, is provided. The system comprises one or more processors 210 configured to:

receive S 110 attenuation data 110 representing an implantable device 120 in an anatomical region, the attenuation data defining X-ray attenuation within the anatomical region;
analyze S 120 the attenuation data 110 to determine an amount of adhesion 130 between the implantable device 120 and a tissue 140 contacting the implantable device; and
output S130 guidance information for the implantable device extraction procedure based on the amount of adhesion 130.

An example of the system 200 is illustrated in Fig. 4. It is noted that the system 200 may also include one or more of: an imaging system for generating the attenuation data 110 that is received in the operation S110, such as for example the CT imaging system 230 illustrated in Fig. 4; a display 220 for displaying the outputted guidance information, and so forth; a patient bed 240; and a user input device configured to receive user input, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the one or more processors of the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A computer-implemented method of providing guidance information for an implantable device extraction procedure, the method comprising:

receiving (S110) attenuation data (110) representing an implantable device (120) in an anatomical region, the attenuation data defining X-ray attenuation within the anatomical region;
analyzing (S120) the attenuation data (110) to determine an amount of adhesion (130) between the implantable device (120) and a tissue (140) contacting the implantable device; and
outputting (S130) guidance information for the implantable device extraction procedure based on the amount of adhesion (130).

**2.** The computer-implemented method according to claim 1, wherein the analyzing (S120) the attenuation data (110) to determine an amount of adhesion (130) between the implantable device (120) and a tissue (140) contacting the implantable device, comprises:

determining a Hounsfield Unit attenuation value of the tissue (140) contacting the implantable device; and determining the amount of adhesion (130) between the implantable device (120) and the tissue (140) contacting the implantable device based on the Hounsfield Unit attenuation value.

**3.** The computer-implemented method according to claim 1, wherein the attenuation data (110) comprises spectral attenuation data, and wherein the spectral attenuation data defines the X-ray attenuation within the anatomical region in a plurality of different energy intervals ($DE_{1..m}$).

**4.** The computer-implemented method according to claim 3, wherein the analyzing (S120) the attenuation data (110) to determine an amount of adhesion (130) between the implantable device (120) and a tissue (140) contacting the implantable device, comprises:

applying a material decomposition algorithm to the spectral attenuation data to identify a type of the tissue contacting the implantable device; and determining the amount of adhesion (130) between the implantable device (120) and the tissue (130) contacting the implantable device based on the type of the tissue.

**5.** The computer-implemented method according to any previous claim, wherein the analyzing (S120) the attenuation data (110) to determine an amount of adhesion (130) between the implantable device (120) and a tissue (140) contacting the implantable device, is performed for a plurality of positions along a length of the implantable device.

**6.** The computer-implemented method according to claim 5, wherein the method comprises determining a total amount of adhesion between the implantable device (120) and a tissue (140) contacting the implantable device, based on the amount of adhesion (130) between the implantable device (120) and the tissue (140) contacting the implantable device at the plurality of positions along the implantable device; and wherein the outputting (S130) guidance information for the implantable device extraction procedure, is based on the total amount of adhesion.

**7.** The computer-implemented method according to any one of claims 1-6, wherein the method further comprises analyzing the attenuation data (110) to determine a value of a risk metric (150), the value of the risk metric representing a risk of damage to a tissue represented in the attenuation data (110) and/or a risk of fracturing the implantable device; and wherein the outputting (S130) guidance information for the implantable device extraction procedure based on the amount of adhesion (130), comprises outputting an indication of the value of the risk metric (150).

**8.** The computer-implemented method according to claim 1, wherein the analyzing (S120) the attenuation data (110) to determine an amount of adhesion (130) between the implantable device (120) and a tissue (140) contacting the implantable device, comprises:

inputting the received attenuation data (110) into a neural network; and predicting, using the neural network, the amount of adhesion (130) between the implantable device (120) and a tissue (140) contacting the implantable device; and wherein the neural network is trained to predict the amount of adhesion between the implantable device and a tissue contacting the implantable device using training attenuation data comprising a plurality of training images representing an implantable device in an anatomical region, and ground truth data comprising ground truth adhesion values corresponding to the training images, the ground truth adhesion values representing the amount of adhesion between the implantable device and a tissue contacting the implantable device.

**9.** The computer-implemented method according to any previous claim, wherein the outputting (S130) guidance information for the implantable device extraction procedure, comprises outputting an indication of one or more of:

the amount of adhesion (130) between the implantable device (120) and a tissue (140) contacting the implantable device; an expected complexity of the implantable device extraction procedure;

an expected duration (160) of the implantable device extraction procedure;
an outcome metric for the implantable device extraction procedure;
a recommended device (170) for use in the implantable device extraction procedure;
a recommended device setting for use in the implantable device extraction procedure;
a recommended procedural step for use in the implantable device extraction procedure.

10. The computer-implemented method according to any previous claim, wherein the analyzing (S120) the attenuation data (110) to determine an amount of adhesion (130) between the implantable device (120) and a tissue (140) contacting the implantable device, comprises quantifying the amount of adhesion between the implantable device and a tissue contacting the implantable device with one or more adhesion classes, or adhesion scores.

11. The computer-implemented method according to any previous claim, wherein the method further comprises:

analyzing the attenuation data (110) to determine a presence of implantable device-induced inflammation in the anatomical region; and
outputting an indication of the presence of the implantable device-induced inflammation.

12. The computer-implemented method according to any previous claim, wherein the method further comprises:

analyzing the attenuation data (110) to determine a stability metric for the implantable device; and
outputting an indication of the stability metric.

13. The computer-implemented method according to claim 12, wherein the method further comprises:

calculating a recommended length of the implantable device (120) to be extracted in the implantable device extraction procedure, the recommended length being calculated based on the amount of adhesion (130) between the implantable device (120) and a tissue (140) contacting the implantable device and/or based on the stability metric and/or the presence of the implantable device-induced inflammation; and
outputting an indication of the recommended length.

14. The computer-implemented method according to any previous claim, wherein the method further comprises segmenting the received attenuation data (110) to identify at least the implantable device (120), prior to the analyzing (S120) the attenuation data (110).

15. The computer-implemented method according to any previous claim, wherein the implantable device (120) comprises a pacemaker lead, or an implantable cardioverter defibrillator lead, or a pacemaker, or an implantable cardioverter defibrillator, or an IVC filter.

FIG. 1

FIG. 2

S110

S120

S130

# FIG. 3

200

230

220

210

240

# FIG. 4

FIG. 5

FIG. 6

Europäisches
Patentamt

European
Patent Office

Office européen
des brevets

# EUROPEAN SEARCH REPORT

Application Number

EP 22 21 3532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/401534 A1 (GRASS MICHAEL [DE] ET AL) 30 December 2021 (2021-12-30) * paragraph [0064] * ----- | 1-15 | INV. A61B6/12 A61B6/00 A61N1/05 |
| A | EP 4 015 035 A1 (KONINKLIJKE PHILIPS NV [NL]) 22 June 2022 (2022-06-22) * paragraph [0029] * ----- | 1-15 | A61B90/00 |
| A | WO 2022/128703 A1 (KONINKLIJKE PHILIPS NV [NL]) 23 June 2022 (2022-06-23) * page 18, line 4 - line 18 * ----- | 1-15 | |

TECHNICAL FIELDS
SEARCHED      (IPC)

A61B
A61N

The present search report has been drawn up for all claims

| Place of search | Date of completion of the search | Examiner |
|---|---|---|
| Munich | 19 July 2023 | Anscombe, Marcel |

EPO FORM 1503 03.82 (P04C01)

## ANNEX TO THE EUROPEAN SEARCH REPORT
## ON EUROPEAN PATENT APPLICATION NO.

EP 22 21 3532

This annex lists the patent family members relating to the patent documents cited in  the above-mentioned European search report.
The members are as contained in the European Patent Office EDP file on
The European Patent Office is in no way liable for these particulars which are merely given for the purpose of information.

19-07-2023

| Patent document cited in search report | | Publication date | Patent family member(s) | | Publication date |
|---|---|---|---|---|---|
| US 2021401534 | A1 | 30-12-2021 | CN | 112739268 A | 30-04-2021 |
| | | | EP | 3636158 A1 | 15-04-2020 |
| | | | EP | 3856037 A1 | 04-08-2021 |
| | | | JP | 2022502130 A | 11-01-2022 |
| | | | US | 2021401534 A1 | 30-12-2021 |
| | | | WO | 2020064518 A1 | 02-04-2020 |
| EP 4015035 | A1 | 22-06-2022 | NONE | | |
| WO 2022128703 | A1 | 23-06-2022 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007034359 A2

**Non-patent literature cited in the description**

- **KEILER, J. et al.** Neointimal fibrotic lead encapsulation - Clinical challenges and demands for implantable cardiac electronic devices. *Journal of Cardiology,* July 2017, vol. 70 (1), 7-17
- **BORNEFALK, H.** Synthetic Hounsfield units from spectral CT data. *Phys Med Biol.,* 07 April 2012, vol. 57 (7), N83-7
- **SAITO, M. et al.** A simple formulation for deriving effective atomic numbers via electron density calibration from dual-energy CT data in the human body. *Medical Physics,* June 2017, vol. 44 (6), 2293-2303
- **BRENDEL, B. et al.** Empirical, projection-based basis-component decomposition method. *Medical Imaging,* 2009
- Physics of Medical Imaging. Proc. of SPIE. vol. 7258, 72583Y
- **ROESSL, E. ; PROKSA, R.** K-edge imaging in X-ray computed tomography using multi-bin photon counting detectors. *Phys Med Biol.,* 07 August 2007, vol. 52 (15), 4679-96
- **SILVA, A. C. et al.** Dual-energy (spectral) CT: applications in abdominal imaging. *RadioGraphics,* 2011, vol. 31 (4), 1031-1046